# EUROPEAN PATENT APPLICATION

(11) **EP 1 921 116 A1**
(43) Date of publication of application: **14.05.2008**
(21) Application number: 06712275.4
(22) Date of filing: 24.01.2006
(51) Int. Cl.: C09B 43/16, B41J 2/01, B41M 5/00, C07D 251/52, C07D 251/70, C09B 33/06, C09D 11/00

(54) **WATER-SOLUBLE AZO COMPOUNDS, INK COMPOSITIONS AND COLORED PRODUCTS**

(30) Priority: 19.08.2005 JP 2005238425
(71) Applicant: Nippon Kayaku Kabushiki Kaisha, Tokyo 102-8172 (JP)
(72) Inventor: TAKAHASHI, Shinjiro, Tokyo 115-8588 (JP); DEJIMA, Yoshiyuki, Tokyo 115-8588 (JP); SHIRASAKI, Yasuo, Saitama-shi Saitama 337-0042 (JP)
(74) Representative: Gille Hrabal Struck Neidlein Prop Roos
(86) International application number: PCT/JP2006/301059
(87) International publication number: WO 2007/020719

(57) **Abstract**

The present invention relates to a water-soluble azo compound represented by the following formula (1)
[KA 1] 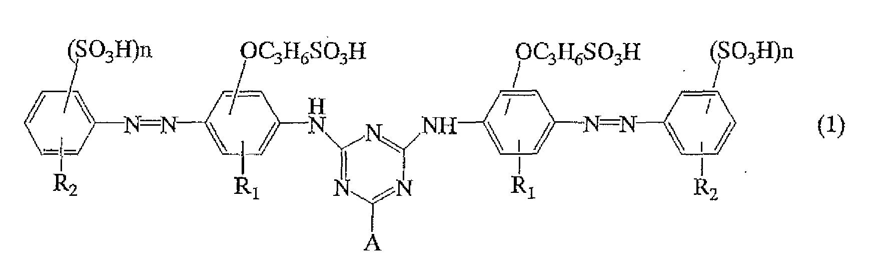 (wherein, A represents a hydroxyl group, a morpholino group, an amino group, an aliphatic amine residue which may have a substituent, aromatic amine residue which may have a substituent, a phenoxy group which may have a substituent or an alkoxy group which may have a substituent, R₁ represents a hydrogen atom or an alkyl group having 1 to 4 carbon atoms, R₂ represents a substituent selected from a hydrogen atom, a halogen atom, a nitro group and a hydroxyl group, n represents an integer number of 1 to 3, respectively) as free acid and an ink composition comprising the same. Said azo compound has hue having high vividness suitable for inkjet recording, said ink composition is excellent in storage stability, and an inkjet recorded article by using this is excellent in fastnesses such as moisture fastness, ozone gas fastness and light fastness.

## Description

### Technical Field

The present invention relates to a water-soluble azo compound, an ink composition comprising the same and a colored article colored therewith.

### Background Art

In the recording method by means of an ink jet printer which is one of typical methods among various color recording methods, various methods for discharging ink have been developed, where ink droplets are generated and adhered onto various record-receiving materials (such as paper, film and cloth) to perform recording. This method has been rapidly prevailing lately and is expected to continue growing remarkably in the future because of such features as quietness with less noise generation due to no direct contact of a recording head with a record-receiving material and as easiness in downsizing, speedup and colorization. Conventionally, as an ink for fountain pens or felt pens and an ink for inkjet recording, water-based inks dissolving a water-soluble dye in an aqueous medium have been used, and in these aqueous inks, a water-soluble organic solvent has been generally added to prevent ink from clogging at a pen tip or an inkjet nozzle. These inks are required to provide recorded images with sufficient density, not to clog at a pen tip or an inkjet nozzle, to dry quickly on record-receiving materials, to bleed less, to have good storage stability, and the like. In addition, recorded images to be formed are required to have fastnesses such as water fastness, moisture fastness, light fastness and gas fastness.

Meanwhile, images or character information on color displays of computers are generally expressed by subtractive color mixing of 4 color inks of yellow (Y), magenta (M), cyan (C) and black (K) for color recording by an ink jet printer. In order to reproduce, as faithfully as possible, images expressed by additive color mixing of red (R), green (G) and blue (B) on CRT (cathode ray tube) displays and the like, as images by subtractive color mixing, it is desired that coloring matters to be used for ink, especially Y, M and C, respectively have hues close to their standards and vividness. In addition, it is required that inks are stable in storage for a long period of time, images printed as mentioned above have a high density, and are excellent in fastnesses such as water fastness, moisture fastness, light fastness, gas fastness. The gas fastness here means durability against the phenomenon that oxidizing gases such as nitrogen oxide gas and ozone gas having oxidizing effect, which exist in the air, reacts with coloring matter (dye) on recording paper or in recording paper having recorded images so as to incur discoloration or fading of printed images. In particular, the ozone gas is regarded as a main causative substance to promote the phenomenon of discoloration of inkjet recorded images. As this phenomenon of discoloration or fading is characteristics of inkjet images, improvement of ozone gas fastness is an important technical challenge in this field. In particular, porous white inorganic substance is used for many of ink receiving layers provided on the surfaces of inkjet professional paper to obtain photo quality in order to dry ink sooner and make bleeding less in high image quality, and on such recording paper, discoloration or fading by the ozone gas is noticeably observed. Along with recent diffusion of digital cameras and color printers, there are more opportunities to print images obtained using digital cameras and the like at home, and fading of images caused by oxidizing gas in the air during storage of printed materials obtained is often considered as a problem.

As an example of the compounds with excellent water-solubility and vividness to be conventionally used as a yellow coloring matter for inkjet, C.I. (color index) direct Yellow 132 can be cited (for example, see Patent Literatures 1 to 3).

[Patent Literature1] JP H11-70729 A
[Patent Literature2] JP 2000-154344 A, Examples A1 to 5
[Patent Literature3] JP 2003-34763 A, Page11 Example 4

### Disclosure of the Invention

### Problems to Be Solved by the Invention

Not all of hue, vividness, light fastness, water fastness, moisture fastness, gas fastness and dissolving stability in C.I. (color index) direct Yellow 132 are satisfactory for use, and development of yellow coloring matter with further improvement on these fastnesses has been required.
Therefore, an object of the present invention is to provide a water-soluble yellow coloring matter (compound) which has high solubility in water and hue and vividness suitable for inkjet recording and which is excellent in water fastness, moisture fastness, light fastness and gas fastness in recorded articles, and an ink composition comprising it.

### Means of Solving the Problems

The inventors of the present invention intensively studied a way to solve the above problems and have found that a water-soluble disazo compound represented by a specific formula and an ink composition comprising it can solve the above problems and completed the present invention.
That is, the present invention relates to;
(1) A water-soluble azo compound represented by the following formula (1)

[KA1]

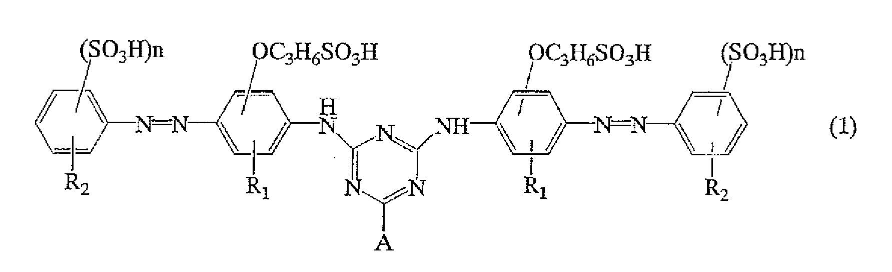

(wherein, A represents a hydroxyl group, an amino group, a morpholino group, an aliphatic amine residue which may have a substituent, an aromatic amine residue which may have a substituent, a phenoxy group which may have a substituent or an alkoxy group which may have a substituent, R₁ represents a hydrogen atom or an alkyl group having 1 to 4 carbon atoms, R₂ represents a substituent selected from a hydrogen atom, a halogen atom, a nitro group and a hydroxyl group, and n represents an integer number of 1 to 3, respectively) as a free acid,
(2) The water-soluble azo compound according to the above (1), wherein R₁ in the formula (1) is a hydrogen atom,
(3) The water-soluble azo compound according to the above (1) or (2), wherein R₂ in the formula (1) is a hydrogen atom,
(4) The water-soluble azo compound according to any one of the above (1) to (3), wherein A in the formula (1) is a group represented by the following formula (2) or (3)

[KA 2]

-NHC₂H₄SO₃H (2)

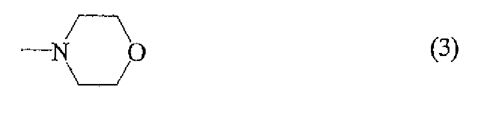

or a hydroxyl group,
(5) An ink composition characterized by comprising the water-soluble azo compound according to any one of the above (1) to (4),
(6) The ink composition according to the above (5), which comprises a water-soluble organic solvent,
(7) The ink composition according to the above (5) or (6), which is for inkjet recording,
(8) An inkjet recording method characterized by that the ink composition according to any one of the above (5) to (7) is used as an ink in an inkjet recording method where ink droplets are discharged responding to recording signals for recording on a record-receiving material,
(9) The inkjet recording method according to the above (8), wherein the record-receiving material is a sheet for information transmission,
(10) The inkjet recording method according to the above (9), wherein the sheet for information transmission is a sheet having an ink receiving layer comprising a porous white inorganic substance,
(11) A colored article colored with the water-soluble azo compound according to any one of the above (1) to (4) or the ink composition according to any one of the above (5) to (7),
(12) The colored article according to the above (11), wherein coloring is performed by an ink jet printer,
(13) An ink jet printer loaded with a container comprising the ink composition according to any one of the above (5) to (7),
(14) The water-soluble azo compound according to the above (1), wherein in the formula (1), R₁ is a hydrogen atom, -OC₃H₆SO₃H is substituted at the meta-position to the azo group, n is 1, and (SO₃H)n is substituted at the 3-position or the 4-position to the azo group,
(15) The water-soluble azo compound according to the above (14), wherein in the formula (1), A is a sulfo C1 to C4 alkylamino group, a morpholino group, an unsubstituted amino group, a carboxyl-substituted phenylamino group, a phenoxy group or a hydroxy group,
(16) The water-soluble azo compound according to the above (15), wherein in the formula (1), A is sulfo C1 to C4 alkylamino group or a morpholino group,
(17) The water-soluble azo compound according to the above (15), wherein in the formula (1), R₂ is a hydrogen atom or a halogen atom,
(18) The water-soluble azo compound according to the above (1) or (14), wherein in the formula (1), A is a sulfoethylamino group or a morpholino group, R₁ is a hydrogen atom and R₂ is a hydrogen atom.

### Effect of the Invention

The water-soluble azo compound represented by the formula (1) of the present invention or a salt thereof (hereinafter, referred to as water-soluble azo compound for short, including a salt) is excellent in solubility in water, has a characteristic that in the process of producing the ink composition, for example, filtration property to membrane filter is good, and provides yellow hue which is very vivid on inkjet recording paper and high in lightness. In addition, the ink composition of the present invention comprising this compound doesn't exhibit crystal precipitate, change in physical property, change in hue, nor the like after storage for a long period of time, and exhibits good storage stability. Further, printed articles by using the ink composition of the present invention as an ink for inkjet recording have ideal hue as yellow hue without selecting the record-receiving material (paper, film or the like), and the ink composition of the present invention can make it possible that photo-like color images are faithfully reproduced on paper. Furthermore, the ink composition of the present invention can provide good fastnesses such as water fastness, moisture fastness, light fastness and gas fastness to recorded articles having surfaces coated with a porous white inorganic substance like inkjet professional paper (film) for photo quality and excellent stability in storage for a long period of time to photo-like recorded images. Thus, the water-soluble azo compound of the formula (1) is extremely useful as a yellow coloring matter for inks, especially an ink for inkjet recording.

### Best Mode for Carrying out the invention

The present invention will be explained more specifically. In this connection, a sulfo group is shown in free acid form unless otherwise specified in the present invention.
The water-soluble azo compound of the present invention is represented by the following formula (1).

[KA3]

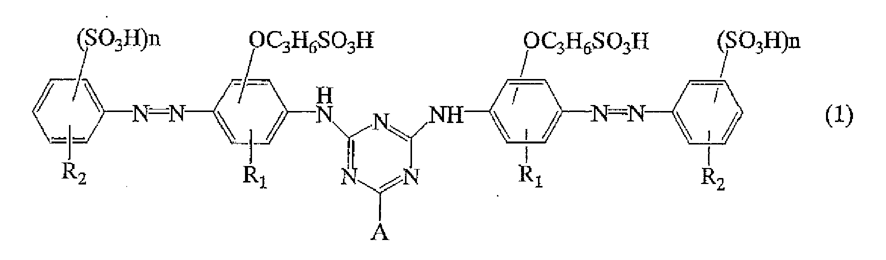

In the formula (1), A represents a hydroxyl group, an amino group, a morpholino group, an aliphatic amine residue which may have a substituent, an aromatic amine residue which may have a substituent, a phenoxy group which may have a substituent or an alkoxy group which may have a substituent.
Unless otherwise specified in the present invention, preferable alkyl, alkoxy or the like includes C1 to C4 alkyl or C1 to C4 alkoxy.
The substituent of the aliphatic amine residue which may have a substituent is preferably a sulfo group, a hydroxyl group or a carboxyl group.
The aliphatic amine residue which may have a substituent preferably includes a mono- or di- (C1 to C4 alkyl) amino group which may have a sulfo group, a hydroxyl group or a carboxyl group as a substituent, and its specific examples include, for example, a 2-sulfoethylamino group, a 2-hydroxyethylamino group, a carboxymethylamino group, a 2-carboxyethylamino group, a 1-carboxyethylamino group, a 1,2-dicarboxyethylamino group, a di(carboxymethyl)amino group and the like, more preferably a 2-sulfoethylamino group.
And the substituents of the aromatic amine residue which may have a substituent are preferably a carboxyl group and a sulfo group.
The aromatic amine residue which may have a substituent includes an unsubstituted anilino group or an anilino group having a carboxyl group or a sulfo group as a substituent, and its specific examples include an anilino group, a 3,5-dicarboxy anilino group, a 4-sulfo anilino group and the like. Further, the substituent of the phenoxy group which may have a substituent is preferably a sulfo group, a carboxyl group, a C1 to C4 acyl group or a hydroxyl group.
Specific examples of the phenoxy group which may have a substituent include a phenoxy group, a 4-sulfophenoxy group, a 4-carboxy phenoxy group, a 4-acetylaminophenoxy group, a 4-hydroxyphenoxy group and the like.
And the substituent of the alkoxy group which may have a substituent is preferably an alkoxy group (preferably C1 to C4 alkoxy group), a hydroxyl group or a carboxyl group.
Specific examples of the alkoxy group which may have a substituent, preferably a C1 to C4 alkoxy group having a C1 to C4 alkoxy group, a hydroxy group or a carboxy group as a substituent, include a methoxyethoxy group, a hydroxyethoxy group, a 3-carboxy propoxy group and the like, respectively.
A is, among them, preferably a morpholino group, a hydroxyl group or an aliphatic amine residue (preferably C1 to C4 alkylamino group) which may have a group selected from a sulfo group or a hydroxyl group as a substituent, more preferably a group selected from the group consisting of a morpholino group, a hydroxyl group and a sulfo C1 to C4 alkylamino group (more preferably sulfoethylamino group), further preferably a morpholino group or a sulfoethylamino group, and most preferably a sulfoethylamino group.
R₁ represents a hydrogen atom or an alkyl group having 1 to 4 carbon atoms and specific examples of the alkyl group having 1 to 4 carbon atoms include a methyl group, an ethyl group, an n-propyl group, an iso-propyl group, an n-butyl group, an iso-butyl group, a sec-butyl group, a tert-butyl group and the like. R₁ is preferably a hydrogen atom among them.
R₂ represents a hydrogen atom, a halogen atom, a nitro group or a hydroxyl group, specific examples of the halogen atom include a chlorine atom, a bromine atom and the like. Among them, R₂ is preferably a chlorine atom or a hydrogen atom, more preferably a hydrogen atom. The substitution position of the sulfopropyloxy group on the benzene ring having substituent R₁ is preferably the 2-position or the 3-position to the substitution position of the azo group on this benzene ring, more preferably the 3-position (meta-position).
The substitution position of the sulfo group on the benzene ring having substituent R₂ is preferably the 2-position, the 3-position or the 4-position to the substitution position of the azo group on this benzene ring, more preferably the 3-position when n = 1, and two substitutions of the 2-position and the 5-position are preferable when n = 2.
n represents an integer number of 1 to 3, preferably 1.

Also, among the water-soluble azo compounds represented by the formula (1) of the present invention, the compound represented by the following formula (4) is preferably cited. And in the formula (4), A₁ represents a morpholino group, a 2-sulfoethylamino group or a hydroxy group, preferably a morpholino group or a 2-sulfoethylamino group, and more preferably a 2-sulfoethylamino group. In this connection, the substitution position of the sulfo group in the formula (4) is preferably the 3-position or the 4-position, more preferably the 3-position, to the azo group.

[KA4]

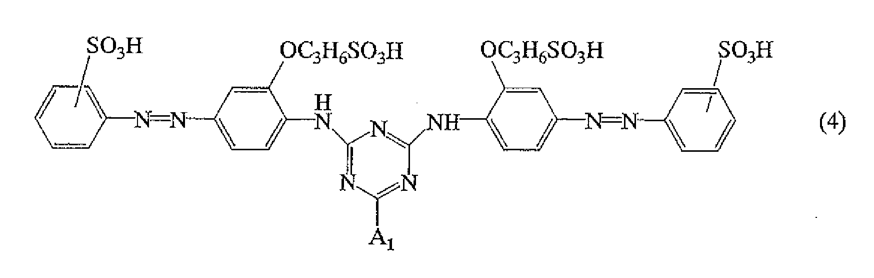

The compound of the formula (1) can have a structure of salt with an inorganic or organic cation. Examples of the salt with an inorganic ion include an alkali metal salt such as a salt with a lithium ion, a salt with a sodium ion or a salt with a potassium ion. Further, examples of the salt with an organic cation include a salt with an ammonium ion represented by the formula (5) and the like.

[KA 5]

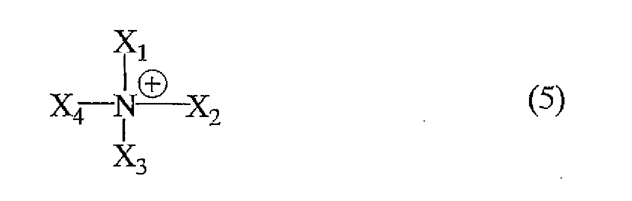

(wherein, each of X₁ to X₄ independently represents a hydrogen atom, an alkyl group ,a hydroxyalkyl group or a hydroxyalkoxyalkyl group.)
As for X₁ to X₄, examples of the alkyl group include a methyl group, an ethyl group and the like. Similarly, examples of the hydroxyalkyl group include a hydroxymethyl group, a hydroxyethyl group, a 3-hydroxypropyl group, a 2-hydroxypropyl group, a 4-hydroxybutyl group, a 3-hydroxybutyl group, a 2-hydroxybutyl group and the like. Further, examples of the hydroxyalkoxyalkyl group include a hydroxyethoxymethyl group, a 2-hydroxyethoxyethyl group, a 3-(hydroxyethoxy)propyl group, a 3-(hydroxyethoxy)butyl group, a 2-(hydroxyethoxy)butyl group and the like.

Preferable salts among the above salts include a sodium salt, a potassium salt, a lithium salt, a monoethanolamine salt, a diethanolamine salt, a triethanolamine salt, a monoisopropanolamine salt, a diisopropanolamine salt, a triisopropanolamine salt, an ammonium salt and the like. Among them, particularly preferable are a lithium salt and a sodium salt.

The salt of the compound of the formula (1) described above can be easily obtained by the following method and the like.
For example, a sodium chloride can be added to a reaction solution before adding 800 parts of methanol in Example1 described later, a solution dissolving a wet cake containing a compound of the formula (1) or its dried one in water for salting out, and then the precipitate can be separated by filtration to obtain a sodium salt of the compound of the formula (1) as a wet cake.
And the wet cake of the obtained sodium salt is dissolved in water and then hydrochloric acid added thereto to adjust the pH from strong acidic to near neutral. The obtained solid is then filtrated. According to the pH, a free acid of the compound of the formula (1) or a mixture of a free acid and a sodium salt where part of the compound of the formula (1) is a sodium salt can be obtained.
Further, while stirring the wet cake of the free acid of the compound of the formula (1) together with water, for example, a potassium hydroxide, a lithium hydroxide, ammonia water, a hydroxide of the formula (5) or the like can be added thereto to make it alkaline to obtain a potassium salt, a lithium salt, an ammonium salt or a quaternary ammonium salt, respectively correspondingly.
Among these salts, particularly preferable are a lithium salt and a sodium salt as describe above.

The water-soluble azo compound represented by the formula (1) of the present invention can be produced, for example, as follows.
That is, for example, the compound of the following formula (A) obtained by referring to the examples described in JP 2004-75719 A is converted to a methyl-co-suffonic acid derivative (B) using a sodium bisulfite and formalin. Subsequently, in the conventional manner, an aromatic amine represented by the following formula (C) is diazotized and subjected to coupling reaction with the above obtained methyl-ω-sulfonic acid derivative of the formula (B) at 0 to 5°C and pH 0 to 2, followed by carrying out hydrolyzation reaction at 60 to 80°C and pH 10.5 to 11.0 to obtain an azo compound having an amino group represented by the following formula (D).
Next, 2 equivalent amount of the obtained azo compound represented by the following formula (D) and 1 equivalent amount of a cyanuric halide, for example, a cyanuric chloride are condensed at a temperature of 20 to 25°C at weakly acidic (typically pH5 to 6) to obtain a condensate represented by the formula (E).
Further, in order to substitute a chlorine atom substituted at the position corresponding to A in the formula (1) in the obtained formula (E) by a group A, for example, a hydroxide ion, ammonia, morpholine, aliphatic amine which may have a substituent, aromatic amine which may have a substituent, phenol which may have a substituent, alcohol which may have a substituent or the like, those compounds are condensed under the conditions of a temperature 75 to 80°C and a pH of 7 to 8 to obtain a water-soluble azo compound represented by the formula (1) of the present invention.
Specific examples of the compound of the following formula (A) include, for example, 2-sulfopropoxyanifine (a compound where R₁ = a hydrogen atom in the formula (A)), 2-sulfopropoxy-5-methylaniline (a compound where R₁ = a methyl in the formula (A)) and the like.
And specific examples of the compound of the following formula (C) include, for example, metanilic acid (3-aminobenzenesulfonic acid, a compound
where R₂ = a hydrogen atom, n = 1, and the substitution position of the sulfo group is the 3-position to the amino group in the formula (C)), sulfanilic acid (4-aminobenzenesulfonic acid, a compound where R₂ = a hydrogen atom, n = 1 and the substitution position of the sulfo group is the 4-position to the amino group in the formula (C)), aniline-2,5-disulfonic acid (a compound where R₂ = a hydrogen atom, n = 2 and the substitution positions of the sulfo group are the 2-position and the 5- position to the amino group in the formula (C)), 3-nitro sulfanilic acid (a compound where R₂ = nitro, n = 1, the substitution position of the sulfo group is the 4-position to the amino group and the substitution position of the nitro group is the 2-position to the same as above in the formula (C)), 4-nitroaniline-2-sulfonic acid (a compound R₂ = nitro, n=1, the substitution position of the sulfo group is the 2-position to the amino group and the substitution position of the nitro group is the 4-position to the same as above in the formula (C)), 3-amino-4-hydroxybenzenesulfonic acid (a compound where R₂ = a hydroxyl group, n = 1, the substitution position of the sulfo group is the 3-position to the amino group and the substitution position of the hydroxyl group is the 6-position to the same as above in the formula (C)), 3-amino-4-chlorobenzenesulfonic acid (a compound where R₂ = a chlorine atom, n = 1, the substitution position of the sulfo group is the 3-position to the amino group and the substitution position of the chlorine atom is the 6-position to the same as above in the formula (C)) and aniline-2-sulfonic acid (a compound where R₂ = a hydrogen atom, n = 1, the substitution position of the sulfo group is the 2-position to the amino group in the formula (C)) available as commercial items. The aliphatic amine which may have a substituent, the aromatic amine which may have a substituent, the phenol which may have a substituent, the alcohol which may have a substituent or the like to be used to substitute a chlorine atom in the formula (E) for a group A can include amines, phenols or alcohols corresponding to the groups mentioned in the above section of the aliphatic amine residue which may have a substituent, the aromatic amine residue which may have a substituent, the phenoxy group which may have a substituent or the alkoxy group which may have a substituent.
The specific example of the water-soluble azo compounds in Table 1 described later can be obtained by producing it in combination of the compound of the formula (A), the compound of the formula (C) and a compound corresponding to group A as cited above as specific examples.

[KA 6]

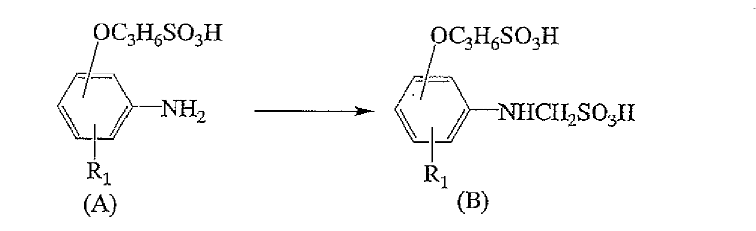

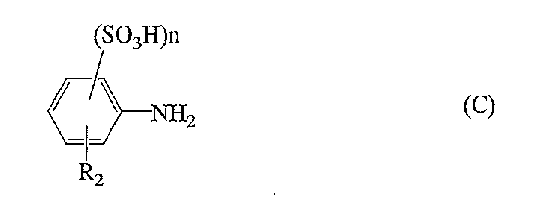

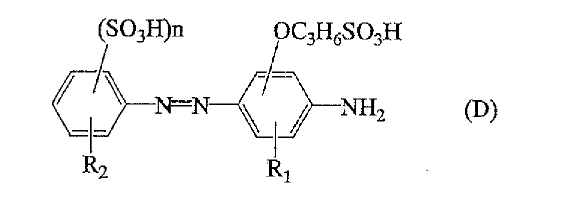

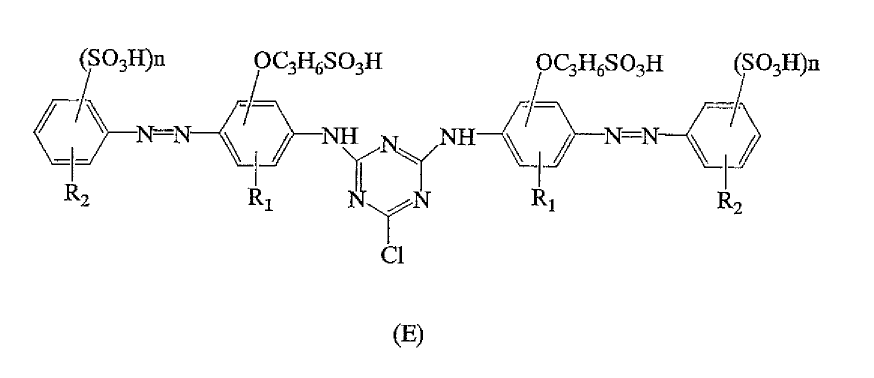

(in the formula (A) through (E), n, R₁ and R₂ have the same meanings as in the formula (1))

Next, one of the water-soluble azo compounds represented by the formula (1) of the present invention can include the compound of the formula (6). In the formula (6), R₁ represents a hydrogen atom or a C1 to C4 alkyl group (preferably methyl group), R₂ represents a hydrogen atom, a nitro group, a hydroxy group or a chlorine atom, n represents 1 or 2, A represents an unsubstituted amino group, a sulfoethylamino group, a 3,5-dicarboxyl anilino group, a morpholino group, a hydroxy group or a phenoxy group. As for the following formula (6), preferable is the compound where R₁ is a hydrogen atom, R2 is a hydrogen atom or a chlorine atom, preferably a hydrogen atom, n is 1, and A is a sulfoethylamino group, a morpholino group or a hydroxy group, preferably a sulfoethylamino group or a morpholino group, more preferably a sulfoethylamino group. Specific examples of the following formula (6) are shown in the following Table 1. In Table 1, the sulfo group, the carboxyl group and the hydroxyl group are shown in free acid form.

[KA 7]

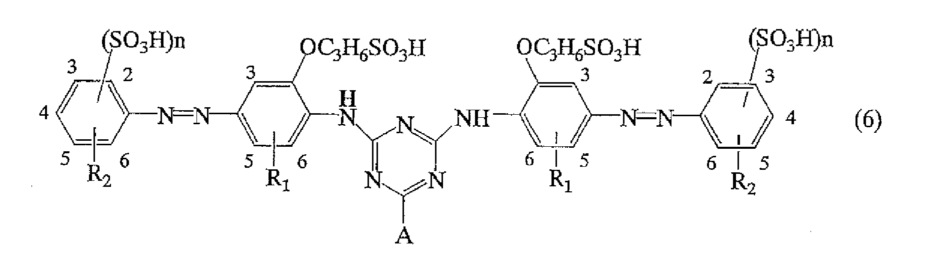

**[Table 1**

| Table 1 Examples of Compounds | | | | | | | |
|---|---|---|---|---|---|---|---|
| Compound Number | -SO₃H | | R₂ | | R₁ | | A |
| | n | Position | Substituent | Position | Substituent | Position | |
| 1 | 1 | 3 | H | - | H | - | -MHC₂H₄SO₃H |
| 2 | 1 | 3 | H | - | H | - | 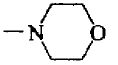 |
| 3 | 1 | 3 | H | - | H | - | -NH₂ |
| 4 | 1 | 3 | H | - | H | - | 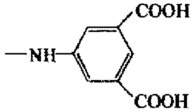 |
| 5 | 1 | 3 | H | - | H | - | 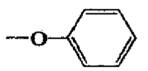 |
| 6 | 1 | 3 | H | - | CH₃ | 5 | -NHC₂H₄SO₃H |
| 7 | 1 | 4 | H | - | H | - | -NHC₂H₄SO₃H |
| 8 | 1 | 4 | H | - | H | - | 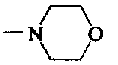 |
| 9 | 1 | 4 | H | - | H | - | -OH |
| 10 | 2 | 2,5 | H | - | H | - | 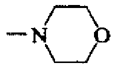 |
| 11 | 1 | 4 | NO₂ | 2 | H | - | -OH |
| 12 | 1 | 2 | NO₂ | 4 | H | - | -OH |
| 13 | 1 | 3 | OH | 6 | H | - | -NHC₂H₄SO₃H |
| 14 | 1 | 3 | Cl | 6 | H | - | -NHC₂H₄SO₃H |
| 15 | 1 | 2 | H | - | H | - | -NHC₂H₄SO₃H |

The water-soluble azo compounds of the present invention are suitable for dyeing natural and synthetic textiles or blended fabrics, and further, these compounds are suitable for manufacturing ink for writing tools and ink compositions for inkjet recording.
Reaction solutions containing a water-soluble azo compound of the formula (1) of the present invention (for example, the reaction solution before adding 800 parts of methanol in Example 1 described later and the like) can be used directly for manufacturing ink composition of the present invention. However, said compound can be also isolated from the reaction solution, dried, for example, spray-dried, and then processed into an ink composition. The ink composition for recording of the present invention contains typically 0.1 to 20 mass%, more preferably 1 to 15 mass%, further preferably 2 to 10 mass% of the water-soluble azo compound of the formula (1) in an aqueous solution. In the ink composition of the present invention, typically 0 to 30 mass%, preferably 5 to 30 mass% of the following water-soluble organic solvent and typically 0 to 10 mass%, preferably 0.05 to 10 mass% of the following ink preparation agent are contained similarly. The rest is water.
The water-soluble azo compound of the formula (1) to be used for preparation of an ink composition preferably has less content of inorganic substances such as a metal cation chloride and sulfuric acid salt, and the whole content of a sodium chloride and a sodium sulfate is approximately not more than 1 mass% in a coloring matter component only as a guide for the content. In order to produce a coloring matter having less inorganic substance, a solution of the bulk powder of a coloring matter may be subjected to desalting treatment by means of a known method per se, for example, using a reverse osmosis membrane and the like.

Specific examples of the water-soluble organic solvent to be used for the ink composition of the present invention include, for example, C1 to C4 alkanol such as methanol, ethanol, n-propanol, isopropanol, n-butanol, isobutanol, secondary butanol or tertiary butanol, carboxylic acid amide such as N,N-dimethylformamide or N,N-dimethylacetamide, lactam such as 2-pyrrolidone, N-methyl-2-pyrrolidone, cyclic urea such as 1,3-dimethylimidazofidin-2-one or 1,3-dimethylhexahydropyrimid-2-one, ketone or keto alcohol such as acetone, methylethylketone or 2-methyl-2-hydroxypentan-4-one, cyclic ether such as tetrahydrofuran or dioxane, monomer, oligomer or polyalkylene glycol or thioglycol having a (C2 to C6) alkylene unit such as ethylene glycol, 1,2- or 1,3-propyleneglycol, 1,2- or 1,4-butyleneglycol, 1,6-hexylene glycol, diethylene glycol, triethylene glycol, tetraethylene glycol, dipropylene glycol, thiodiglycol, polyethylene glycol or polypropylene glycol, polyol (triol) such as glycerine or hexane-1,2,6-triol, polyhydric alcohol (C1 to C4) alkyl ether such as ethylene glycol monomethyl ether or ethylene glycol monoethyl ether, diethylene glycol monomethyl ether or diethylene glycol monoethyl ether or diethylene glycol monobutyl ether (butylcarbitol) or triethylene glycol monomethyl ether or triethylene glycol monoethyl ether, γ-butyrolactone, dimethylsulfoxide and the like. These water-soluble organic solvents are used alone or in mixture. Among them, preferable are isopropanol, glycerine, butylcarbitol, 2-pyrrolidone, N-methyl-2-pyrrolidone, mono-, di- or tri-ethylene glycol, dipropylene glycol, more preferably 2-pyrrolidone, N-methyl-2-pyrrolidone, isopropanol, glycerine, butylcarbitol or diethylene glycol.

The ink preparation agents mean various additives other than water and the azo compound of the formula (1) to be used in ink preparation, and those include, for example, an antiseptic and fungicide, a pH modifier, a chelating agent, a rust-preventive agent, a water-soluble UV absorbing agent, a water-soluble polymer compound, a dye dissolving agent, a surfactant and the like. The antiseptic and fungicide includes, for example, each compound of organic sulfur, organic nitrogen sulfur, organic halogen, haloallylsulfone, iodopropargyl, N-haloalkylthio, nitrile, pyridine, 8-oxyquinoline, benzothiazole, isothiazoline, dithiol, pyridine oxide, nitropropane, organic tin, phenol, quaternary ammonium salt, triazine, thiadiazine, anilide, adamantane, dithiocarbamate, brominated indanone, benzyl bromoacetate, inorganic salt compounds. The organic halogen compound includes, for example, sodium pentachlorophenol, the pyridine oxide compound includes, for example, sodium 2-pyridinethiol-1-oxide, the isothiazoline compound includes, for example, 1,2-benzisathiazolin-3-one, 2-n-octyl-4-isothiazolin-3-one, 5-chloro-2-methyl-4-isothiazolin-3-one, 5-chloro-2-methyl-4-isothiazolin-3-one magnesium chloride, 5-chloro-2-methyl-4-isothiazolin-3-one calcium chloride, 2-methyl-4-isothiazolin-3-one calcium chloride and the like, respectively. Other antiseptic and fungicides include sodium sorbate, sodium benzoate and the like.

As the pH modifier, any substance can be used as long as it can control the pH of the ink typically in the range of 7.0 to 11.0 without any adverse effects on the ink composition to be prepared. Examples of the pH modifier to be used include, for example, alkanolamines such as diethanolamine and triethanolamine, alkali metal hydroxides such as lithium hydroxide, sodium hydroxide and potassium hydroxide, ammonium hydroxide (ammonia water), alkali metal carbonates such as lithium carbonate, sodium carbonate and potassium carbonate, or the like. The chelating agent includes, for example, sodium ethylenediamine tetraacetate, sodium nitrilotriacetate, sodium hydroxyethylethylenediamine triacetate, sodium diethylenetriamine pentaacetate, sodium uracil diacetate and the like. The rust-preventive agent includes, for example, acidic sulfite salt, sodium thiosulfate, ammonium thioglycolate, diisopropylammonium nitrite, pentaerythritol tetranitrate, dicyclohexylammonium nitrite and the like.

The water-soluble UV absorbing agent includes, for example, sulfonated benzophenone, sulfonated benzo triazole or the like. The water-soluble polymer compound includes, for example, polyvinyl alcohol, cellulose derivatives, polyamines, polyimines and the like. The dye dissolving agent includes, for example urea, ε-caprolactam, ethylene carbonate and the like. The surfactant includes, for example, anionic surfactants, amphoteric surfactants, cationic surfactants, nonionic surfactants and the like. The anionic surfactant includes alkyl sulfocarboxylate, α-olefin sulfonate, polyoxyethylene alkyl etheracetate, n-acylamino acid and salt thereof, n-acylmethyltaurine salt, rosin acid soap, castor oil sulfate, lauryl alcohol sulfate, alkylphenol phosphate, alkyl phosphate, alkylallylsulfonate, diethyl sulfosuccinate, diethylhexyl sulfosuccinate, dioctyl sulfosuccinate and the like. The cationic surfactant includes 2-vinylpyridine derivatives, poly 4-vinyl pyridine derivative and the like.

The amphoteric surfactant includes, for example, lauryldimethylaminoacetic acid betaine, 2-alkyl-N-carboxymethyl-N-hydroxyethylimidazolinium betaine, coconut oil fatty acid amide propyldimethylaminoacetic acid betaine, polyoctylpolyaminoethylglycine, imidazoline derivatives and the like. The nonionic surfactant includes ethers such as polyoxyethylene nonylphenyl ether, polyoxyethylene octylphenyl ether, polyoxyethylene dodecylphenyl ether, polyoxyethylene octylphenyl ether, polyoxyethylene oleyl ether, polyoxyethylene lauryl ether and polyoxyethylene alkyl ether, esters such as polyoxyethylene oleic acid, polyoxyethylene oleic acid ester, polyoxyethylene distearate, sorbitan laurate, sorbitan monostearate, sorbitan monooleate, sorbitan sesquioleate, polyoxyethylene monooleate, polyoxyethylene stearate, acetylene glycols such as 2,4,7,9-tetramethyl-5-decyne-4,7-diol, 3,6-dimethyl-4-octyne-3,6-diol and 3,5-dimethyl-1-hexin-3-ol (for example, Surfynol 104, 104PG50, 82, 465, Olfine STG and the like, manufactured by Nissin Chemical Industry Co., Ltd.), and the like.
These ink preparation agents are used alone or in mixture, respectively.

The ink composition of the present invention can be produced by dissolving the water-soluble azo compound represented by the formula (1) in water, if necessary, together with a water-soluble organic solvent, the above ink preparation agents and the like. When a reaction solution containing the water-soluble azo compound of the formula (1) is directly used for producing the ink composition, it has preferably a less content of inorganic substances such as metal cation chlorides, sulfuric acid salts and the like, and the content is, only as a guide, approximately not more than 1 mass% per a bulk powder of coloring matter contained in the reaction solution as described above. In order to produce a coloring matter with less inorganic substance, the reaction solution, for example, can be subjected to desalting treatment by a known method per se using a reverse osmosis membrane.

The order of dissolving the ingredients in the above producing method is not particularly limited. The water-soluble azo compound represented by the formula (1) may be dissolved in water and/or a water-soluble organic solvent in advance, and then an ink preparation agent may be added and dissolved therein; or the water-soluble azo compound represented by the formula (1) may be dissolved in water, and then a water-soluble organic solvent and an ink preparation agent may be added and dissolved therein. The order may be different from this, and a water-soluble organic solvent and an ink preparation agent are added to the reaction solution of the water-soluble azo compound represented by the formula (1) or a liquid of the water-soluble azo compound represented by the formula (1) subjected to desalting treatment using a reverse osmosis membrane, for producing the ink composition. Water to be used for preparation of the ink composition of the present invention has preferably less impurities, such as ion-exchanged water, distilled water or the like. In addition, after preparing the ink composition, foreign substances may be removed by carrying out microfiltration, if necessary, using membrane filters and the like, and microfiltration is preferably carried out particularly in the case of using it as an ink composition for an ink jet printer. The pore size of filter to be used for carrying out microfiltration is typically 1 micron to 0.1 micron, preferably 0.8 microns to 0.2 microns.

The ink composition containing the water-soluble azo compound of the present invention is suitable for impress printing, copying, marking, writing, drafting, stamping and recording (printing), especially for inkjet recording. In this case, high quality articles printed yellow which have good fastnesses against water, light, ozone and friction.

The colored article of the present invention is an article colored with the compound of the present invention. The materials to be colored are not limited, including e.g. paper, fiber and cloth (cellulose, nylon, wool and the like), leather, substrates for color filters and the like, but not limited thereto. The coloring method includes, for example, printing methods such as dip dyeing, textile printing, screen printing, methods by ink jet printers and the like, preferably methods by ink jet printers.

There is an ink jet printer where two kinds of inks, a high concentration ink and a low concentration ink, are loaded for the purpose of supplying high resolution images. In that case, an ink composition with a high concentration and an ink composition with a low concentration may be manufactured respectively using the water-soluble azo compound represented by the formula (1) of the present invention, and then they can be used for an ink set. Otherwise, the ink composition containing the water-soluble azo compound represented by the formula (1) may be used for either of the inks. Further, the water-soluble azo compound represented by the formula (1) of the present invention and a known yellow coloring matter may be used in combination. The water-soluble azo compound represented by the formula (1) can be used in combination with other colors for the purpose of adjusting the hue of another color, for example, black ink or for the purpose of preparing a red ink or a green ink by mixing with a magenta coloring matter or a cyan coloring matter.

Record-receiving materials (media) on which the inkjet recording method of the present invention can be applied include, for example, sheet for information transmission, textile, leather and the like. The sheet for information transmission is preferably subjected to surface treatment, specifically provided with an ink receiving layer on the substrate of paper, film or the like. The ink receiving layer can be provided, for example, by impregnating or coating a cation polymer on the above substrate, or by coating on the surface of the above substrates, a porous white inorganic substance which can absorb coloring matter in the ink, such as porous silica, aluminasol or special ceramics, together with a hydrophilic polymer such as polyvinyl alcohol or polyvinylpyrrolidone. Such paper as provided with an ink receiving layer is typically called inkjet professional paper (film) or glossy paper (film), and commercially available, for example, under the names of Pictorico (trade name, manufactured by Asahi Glass Co., Ltd.), Professional Photopaper, Super Photopaper, Matte Photopaper (all are trade names, manufactured by Canon Inc.), Photo Paper (Glossy), Photo Matte Paper and Super Fine Glossy Film (all are trade names, manufactured by SEIKO-EPSON CORPORATION), Premium Plus Photo Paper, Premium Glossy Film and Photo Paper (all are trade names, manufactured by Hewlett Packard Japan, Ltd.), PhotoLikeQP (trade name, manufactured by KONIKA Corporation) and the like. In this connection, plain paper is obviously used.

Among them, it is known that ozone gas develops discoloration or fading of images recorded particularly on a record-receiving material coated with a porous white inorganic substance on the surface, but the ink composition of the present invention imparts superior recorded images with less discoloration or fading in the case of recording on such record-receiving materials due to its excellent gas fastness.

In order that recording is performed on a record-receiving material by the inkjet recording method of the present invention, for example, a container filled with the above ink composition is set in the predefined position of an inkjet printer to record on a record-receiving material in a general way. In the inkjet recording method of the present invention, the yellow ink composition of the present invention can be used in combination with a magenta ink composition, a cyan ink composition, if necessary, a green ink composition, a blue (or violet) ink composition, a red ink composition, a black ink composition and the like. In this case, each of the color ink compositions is filled into each of the containers, and then the containers are loaded in the predefined positions of an ink jet printer for use. Examples of the ink jet printer to be used include printers, for example, a piezo inkjet printer utilizing mechanical vibration and a bubble-jet printer (registered trademark) utilizing bubbles generated by heating, and the like.

The ink composition of the present invention exhibits vivid yellow and has high color definition and suitable hue for the inkjet recording method, particularly on glossy paper for inkjet. In addition, it is characterized by very high fastness of the recorded images. The ink composition of the present invention is not precipitated nor separated during storage. Further, the ink composition of the present invention does not cause clogging of injectors (ink heads) when used for inkjet recording. The ink composition of the present invention exhibits no change in its physical property under recirculation for a relatively long period of time interval by a continuous inkjet printer or in intermittent use by an on-demand inkjet printer.

Hereinafter, the present invention will be further specifically explained by Examples. In this connection, "parts" and "%" in Examples are based on mass unless otherwise specified.
And each Amax of the synthesized compounds is shown as the measured value in an aqueous solution of pH 7 to 8. In addition, the obtained compounds of the formulas (7) to (10) are shown in free acid form for convenience, but in the present examples, the compounds of the formulas (7) to (10) were obtained as a mixture of free acid and a sodium salt. As described above, however, an alkali metal salt and the like other than free acid or a sodium salt can be easily obtained by using an appropriate method and the present invention is not limited to the present examples.

### Example 1

While adjusting the pH at 6 with a sodium hydroxide, 34.6 parts of 3-aminobenzenesulfonic acid was dissolved in 210 parts of water, and 14.8 parts of sodium nitrite was added thereto. This solution was added dropwise in 510 parts of 5% hydrochloric acid of 5 to 10°C over 30 minutes, and then stirred at no higher than 10°C for 1 hour to carry out diazotization reaction. Next, 46.2 parts of 2-sulfopropoxyaniline was dissolved in 130 parts of water while adjusting the pH at 5 with a sodium hydroxide and turned into methyl-ω-suffonic acid derivatives in a conventional manner using 21.8 parts of sodium bisulfite and 18.0 parts of 35% formalin, which derivatives were then charged into the above produced diazonium salt and stirred at 0 to 5°C and pH 0 to 2 for 2 hours. The reaction solution was adjusted at pH 11 with a sodium hydroxide and then stirred at 65 to 70°C for 5 hours while maintaining the same pH, and further subjected to salting out with 240 parts of a sodium chloride to obtain 270 parts of an azo compound having an amino group as a wet cake. Next, 0.14 parts of LEOCOL TD90 (surfactant, manufactured by Lion Corporation) was added in 250 parts of ice water and stirred violently, and 13.8 parts of a cyanuric chloride was added therein and stirred at 0 to 5°C for 30 minutes. Subsequently, the resulting suspension was added dropwise, over 30 minutes, into the solution obtained with 270 parts of the above obtained wet cake (azo compound having an amino group) and 400 parts of water. After completion of the dropwise addition, it was stirred at pH 5 to 6 and 20 to 25°C for 2 hours. After that, 11.2 parts of taurine was charged to the resulting reaction solution and stirred at pH 7 to 8 and 75 to 80°C for 3 hours. After the resulting reaction solution was cooled to 20 to 25°C, 800 parts of methanol was charged into this reaction solution and stirred at 20 to 25°C for 1 hour and the precipitate was separated by filtration to obtain 95.0 parts of a wet cake. This wet cake was dried by a hot air dryer (80°C) to obtain 60.0 parts of a water-soluble azo compound (λmax 389 nm) of the present invention represented by the following formula (7).

[KA 8]

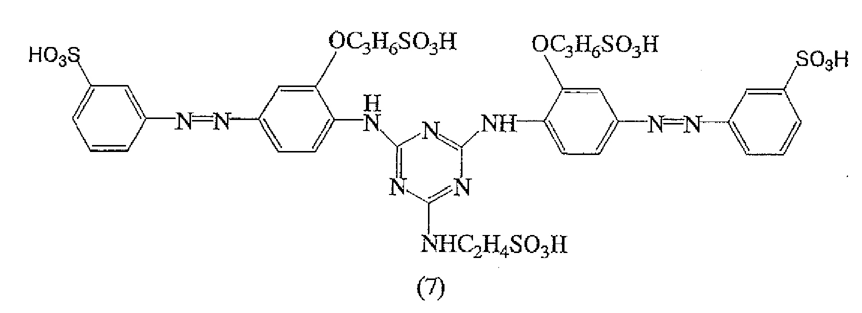

### Example 2

In the same manner as in Example 1 except that 7.8 parts of morpholine was used instead of 11.2 parts of taurine in Example 1, 58.0 parts of a water-soluble azo compound (λmax 391 nm) represented by the following formula (8) of the present invention was obtained.

[KA 9] 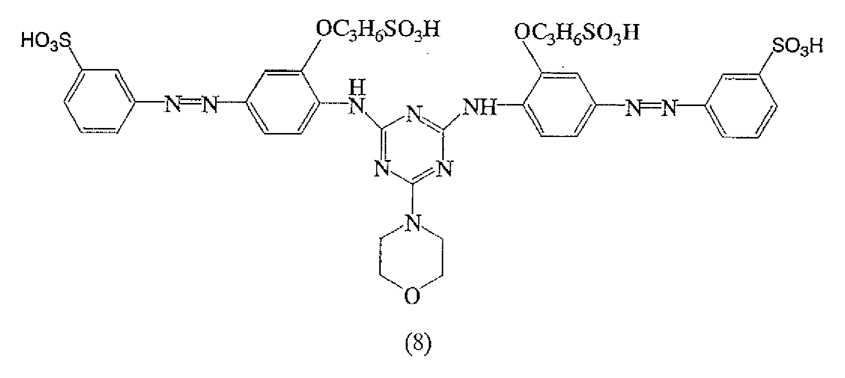 Example 3

In the same manner as in Example 1 except that 34.6 parts of 4-aminobenzenesulfonic acid was used instead of 34.6 parts of 3-aminobenzenesulfonic acid in Example 1, 62.0 parts of a water-soluble azo compound (λmax 394 nm) of the present invention represented by the formula (9) of the present invention was obtained.

[KA 10] 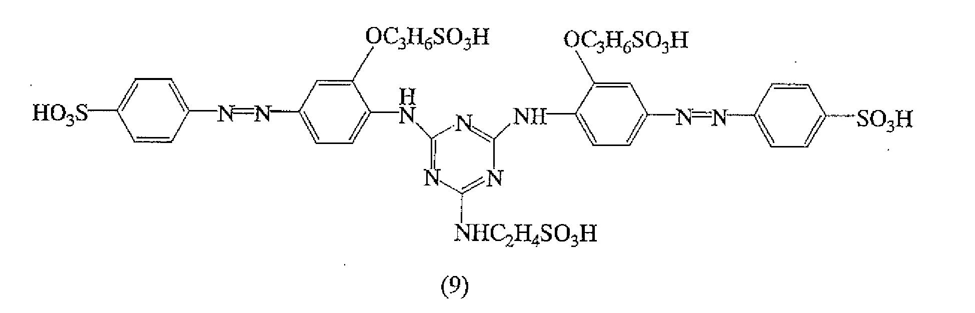 Example 4

In the same manner as in Example 2 except that 34.6 parts of 4-aminobenzenesulfonic acid was used instead of 34.6 parts of 3-aminobenzenesulfonic acid in Example 2, 57.0 parts of a water-soluble azo compound of the present invention represented by the formula (10) was obtained.

[KA 11]

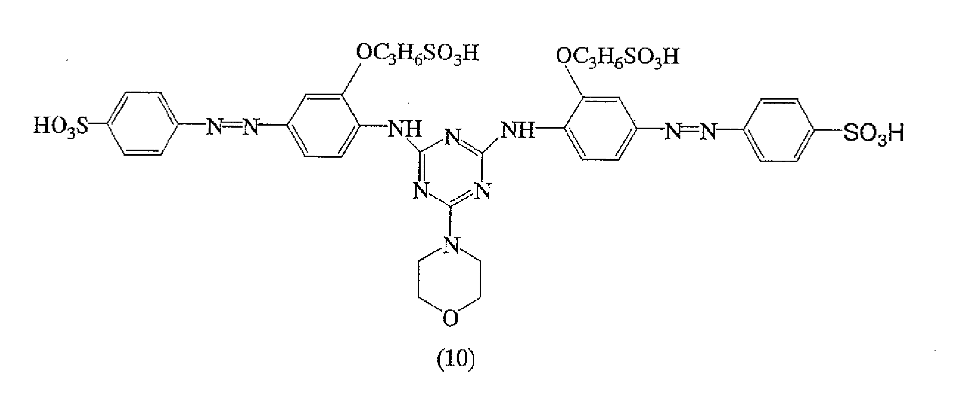

### Examples 5 to 8

### (A) Preparation of Ink

Each of the azo compounds of the present invention obtained in the above Examples1, 2, 3 and 4 was mixed at the composition ratio shown in Table 2 to obtain each of the ink compositions of the present invention, and foreign substances were removed therefrom respectively by filtration with 0.45 µm membrane filters. In this connection, ion-exchanged water was used as water and the pH of the ink composition was adjusted at pH = 7 to 9 with 28% ammonia water, and then water was added thereto so that the total amount was 100 parts. The ink compositions obtained by using the azo compounds obtained in Example 1, Example 2, Example 3 and Example 4 are respectively for Example 5, Example 6, Example 7 and Example 8.

**[Table 2]**

| Table 2 (Composition ratio of ink composition) | |
|---|---|
| Each azo compound obtained in Example 1 to Example 4 | 5.0 parts |
| Glycerine | 5.0 parts |
| Urea | 5.0 parts |
| N-methyl-2-pyrrolidone | 4.0 parts |
| Isopropyl alcohol | 3.0 parts |
| Butyl carbitol | 2.0 parts |
| Surfynol 104PG50 (Note) | 0.1 part |
| 28% ammonia water + water | 75.9 parts |
| Total | 100.0 parts |

| | |
|---|---|
| (Note) Trade name, an acetylene glycol nonionic surfactant, manufactured by Nissin Chemical Industry Co., Ltd. | |

As a control for comparison, an ink composition for comparison was prepared at the composition ratio of Table 3 using C.I. Direct Yellow 132 which is widely used as a yellow coloring matter for inkjet (Comparative Example 1).

**[Table 3]**

| Table 3 (Composition ratio of ink composition for comparison) | |
|---|---|
| C.I. Direct Yellow 132 | 3.0 parts |
| Glycerine | 5.0 parts |
| Urea | 5.0 parts |
| N-methyl-2-pyrrolid one | 4.0 parts |
| Isopropyl alcohol | 3.0 parts |
| Butyl carbitol | 2.0 parts |
| Surfynol 104PG50 (the above note) | 0.1 part |
| 28% ammonia water + water | 77.9 parts |
| Total | 100.0 parts |

### (B) Inkjet Printing

Using an ink jet printer (trade name: Pixus 860i, manufactured by Canon Inc.), inkjet recording was performed on two kinds of paper, glossy paper 1 (Professional Photopaper PR-101, manufactured by Canon Inc.) and glossy paper 2 (trade name: Photo Paper (Glossy) KA450PSK, manufactured by SEIKO-EPSON CORPORATION), having an ink receiving layer containing a porous white inorganic substance. In inkjet recording, such an image pattern was made that several gradations of reflection density can be obtained and yellow printed article were obtained. Moisture fastness test was carried out using a print having unprinted part and printed part, and in light fastness test and ozone gas fastness test, measurement of reflection density was carried out on the part of a printed article which had had the reflection density D value nearest to 1 before the test. In this connection reflection density was measured using a colorimetric system "GRETAG SPM50, manufactured by Gretag Macbeth AG".

### (C) Moisture fastness test for recorded image

The test pieces printed on glossy paper 1 and glossy paper 2 were left at 50°C and 90% RH for 7 days by using a thermo-hygrostat (manufactured by Ohken. Co., Ltd) and bleeding of the coloring matter (dye) from the printed part to the unprinted part is judged by visual observation. The results are shown in Table 4. The evaluation criteria are as follows.
○ Bleeding of the coloring matter to the unprinted part is hardly observed.
Δ Bleeding of the coloring matter to the unprinted part is slightly observed.
× Bleeding of the coloring matter to the unprinted part is largely observed.

### (D) Xenon light fastness test of recorded image

The test pieces printed on glossy paper 1 and glossy paper 2 were placed on the holder together with a glass plate having a thickness of 2 mm through an air layer between them and irradiated at an illuminance of 0.36 W/m² for 50 hours, using a xenon weatherometer Ci4000 (trade name, manufactured by ATRAS Electric Devices Co.). After the test, reflection density was measured using the colorimetric system. After the measurement, residual rates of the coloring matter were calculated by (reflection density after the test / reflection density before the test) x 100 (%) to evaluate according to 3 scales.

| | |
|---|---|
| Residual rate of coloring matter is not less than 85% | ○ |
| Residual rate of coloring matter is not less than 75% and less than 85% | Δ |
| Residual rate of coloring matter is less than 75% | × |

The results are shown in Table 4.

### (E) Ozone gas fastness test of recorded image

The test pieces printed on glossy paper 1 and glossy paper 2 were left for 3 hours under the circumstances of an ozone concentration of 40ppm, a humidity of 60% RH and a temperature of 24°C, using an ozone weatherometer (trade name, manufactured by Suga Test Instruments Co., Ltd.). After the test, reflection density is measured using the above colorimetric system. After the measurement, residual rates of the coloring matters were calculated by (reflection density after the test / reflection density before the test) x 100 (%) to evaluate according to 3 scales.

| | |
|---|---|
| Residual rate of coloring matter is not less than 65% | ○ |
| Residual rate of coloring matter is not less than 55% and less than 65% | Δ |
| Residual rate of coloring matter is less than 55% | × |

The results are shown in Table 4.

### (F) Solubility test

On the azo compounds used for the ink compositions of Examples 5 to 8 (the compounds (Na salts) obtained in Example 1 to Example 4), solubility to water was tested. Ion-exchanged water was used as water, and the test was carried out at around pH 8 and room temperature (about 25°C). Solubility was evaluated under the following evaluation criteria.

| | |
|---|---|
| Solubility is high compared with C.I. Direct Yellow 132 | ○ |
| Solubility is equivalent to C.I. Direct Yellow 132 | Δ |
| Solubility is low compared with C.I. Direct Yellow 132 | × |

The results are shown in the sections of Examples 5 (azo compound of Example 1), 6 (azo compound of Example 2), 7 (azo compound of Example 3) and 8 (azo compound of Example 4) in Table 4.

**[Table 4]**

| Table 4: The results of the tests | | | | |
|---|---|---|---|---|
| Solubility | | Moisture fastness | Ozone gas fastness | Light fastness |
| Example 5 ○ | | | | |
| | (Glossy paper 1) | ○ | ○ | ○ |
| | (Glossy paper 2) | ○ | ○ | ○ |
| Example 6 ○ | | | | |
| | (Glossy paper 1) | ○ | ○ | ○ |
| | (Glossy paper 2) | ○ | ○ | ○ |
| Example 7 ○ | | | | |
| | (Glossy paper 1) | ○ | ○ | ○ |
| | (Glossy paper 2) | ○ | ○ | ○ |
| Example 8 ○ | | | | |
| | (Glossy paper 1) | ○ | ○ | ○ |
| | (Glossy paper 2) | ○ | ○ | ○ |
| Comparative Example 1 - | | | | |
| | (Glossy paper 1) | ○ | Δ | Δ |
| | (Glossy paper 2) | × | ○ | ○ |

As clear from the results of Table 4, the compound of Comparative Example1 has a residual rate of coloring matter of no less than 55% and less than 65% in the ozone gas fastness test using glossy paper 1, and no less than 75% and less than 85% in the light fastness test, resulting in that it has a problem in terms of these fastnesses. Further, bleeding of coloring matter to the unprinted part was largely observed in the moisture fastness test using glossy paper 2 and it is found that it also has a problem in terms of moisture fastness.
Compared with this, for the ink compositions of Example 5 to Example 8 (the ink compositions of the present invention), even when any of the glossy papers were used, the residual rate of coloring matter was no less than 65% in the ozone gas fastness test and no less than 85% in the light fastness test, and bleeding of coloring matter to the unprinted part was hardly observed in the moisture fastness test, showing high fastnesses in all the tests. In addition, the present compound also showed results exceeding the compound of Comparative Example 1 in the solubility test.
Judging from the above results, it is clear that the water-soluble azo compound of the present invention is a compound suitable to prepare ink compositions particularly for inkjet recording and very useful as a yellow coloring matter for inkjet ink.

### Industrial Applicability

The water-soluble azo compound represented by the formula (1) of the present invention has excellent solubility to water and good filtration, e.g. through membrane filters, in the process of producing ink compositions; and in addition, the obtained ink composition doesn't exhibit crystal precipitate, change in physical property, change in hue, nor the like after storage for a long period of time, and expresses good storage stability. Further, when recording is performed on inkjet professional paper (film) and the like using said ink composition by an inkjet printer, the recorded article obtained has excellence in fastnesses such as water fastness, moisture fastness, light fastness and gas fastness and the recorded images and the like have excellent storage stability for a long period of time. Therefore, the water-soluble azo compound of the formula (1) is extremely useful as a yellow coloring matter for ink, particularly for ink for inkjet recording.

## Claims

1. A water-soluble azo compound represented by the following formula (1)
[KA 1] 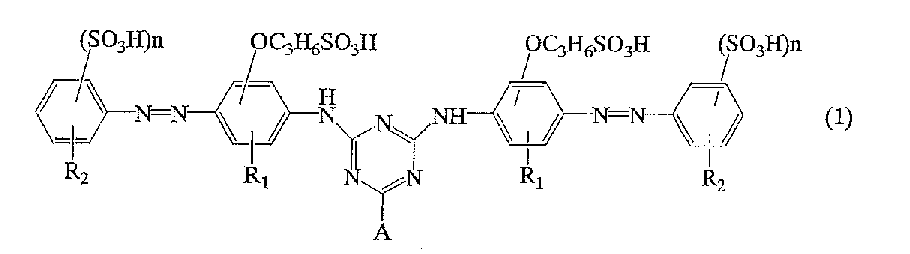 (wherein, A represents a hydroxyl group, an amino group, a morpholino group, an aliphatic amine residue which may have a substituent, an aromatic amine residue which may have a substituent, a phenoxy group which may have a substituent or an alkoxy group which may have a substituent, R₁ represents a hydrogen atom or an alkyl group having 1 to 4 carbon atoms, R₂ represents a substituent selected from a hydrogen atom, a halogen atom, a nitro group and a hydroxyl group, and n represents an integer number of 1 to 3, respectively) as a free acid.

2. The water-soluble azo compound according to Claim 1, wherein R₁ in the formula (1) is a hydrogen atom.

3. The water-soluble azo compound according to Claim 1 or 2, wherein R₂ in the formula (1) is a hydrogen atom.

4. The water-soluble azo compound according to any one of Claims 1 to 3, wherein A in the formula (1) is a group represented by the following formula (2) or (3)
[KA 2]
NHC₂H₄SO₃H (2) 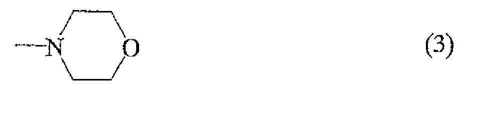
or a hydroxyl group.

5. An ink composition **characterized by** comprising the water-soluble azo compound according to any one of Claims 1 to 4.

6. The ink composition according to Claim 5, which comprises a water-soluble organic solvent.

7. The ink composition according to Claim 5 or 6, which is for inkjet recording.

8. An inkjet recording method **characterized by** that the ink composition according to any one of Claims 5 to 7 is used as an ink in an inkjet recording method where ink droplets are discharged responding to recording signals for recording on a record-receiving material.

9. The inkjet recording method according to Claim 8, wherein the record-receiving material is a sheet for information transmission.

10. The inkjet recording method according to Claim 9, wherein the sheet for information transmission is a sheet having an ink receiving layer comprising a porous white inorganic substance.

11. A colored article colored with the water-soluble azo compound according to any one of Claims 1 to 4 or the ink composition according to any one of Claims 5 to 7.

12. The colored article according to Claim 11, wherein coloring is performed by an ink jet printer.

13. An ink jet printer loaded with a container comprising the ink composition according to any one of Claims 5 to 7.

14. The water-soluble azo compound according to Claim 1, wherein in the formula (1), R₁ is a hydrogen atom, -OC₃H₆SO₃H is substituted at the meta-position to the azo group, n is 1, and (SO₃H)n is substituted at the 3-position or the 4-position to the azo group.

15. The water-soluble azo compound according to Claim 14, wherein in the formula (1), A is a sulfo C1 to C4 alkylamino group, a morpholino group, an unsubstituted amino group, a carboxyl-substituted phenylamino group, a phenoxy group or a hydroxy group.

16. The water-soluble azo compound according to Claim 15, wherein in the formula (1), A is sulfo C1 to C4 alkylamino group or a morpholino group.

17. The water-soluble azo compound according to Claim 15, wherein in the formula (1), R₂ is a hydrogen atom or a halogen atom.

18. The water-soluble azo compound according to Claim 1 or 14, wherein in the formula (1), A is a sulfoethylamino group or a morpholino group, R₁ is a hydrogen atom and R₂ is a hydrogen atom.
